# EUROPEAN PATENT SPECIFICATION

(11) **EP 2 022 861 B1**
(45) Date of publication and mention of the grant of the patent: **06.11.2013**
(21) Application number: 07743388.6
(22) Date of filing: 15.05.2007
(51) Int. Cl.: C12Q 1/68, C12M 1/00, C12M 1/26, C12M 3/00, G01N 33/15, C12N 15/09

(54) **METHOD FOR DETECTING DISEASE-RELATED MARKER USING GASTRIC MUCOSAL LAVAGE FLUID**
VERFAHREN ZUM NACHWEIS EINES MARKERS IN VERBINDUNG MIT EINER KRANKHEIT UNTER VERWENDUNG VON MAGENSCHLEIMHAUT-LAVAGEFLÜSSIGKEIT
PROCÉDÉ DE DÉTECTION D'UN MARQUEUR D'UNE MALADIE À L'AIDE D'UN FLUIDE DE LAVAGE DES MUQUEUSES GASTRIQUES

(30) Priority: 15.05.2006 JP 2006134878
(43) Date of publication of application: 11.02.2009
(73) Proprietor: Sapporo Medical University, Sapporo-shi, Hokkaido 060-0061 (JP); St. Marianna University School of Medicine, Kawasaki-shi, Kanagawa 216-8511 (JP); Hitachi Chemical Company, Ltd., Tokyo 163-0449 (JP)
(72) Inventor: WATANABE, Yoshiyuki, Yokohama-shi, Kanagawa 227-0046 (JP); TOYOTA, Minoru, Sapporo-shi, Hokkaido 060-8556 (JP); IMAI, Kohzo, Sapporo-shi, Hokkaido 060-8556 (JP); SHINOMURA, Yasuhisa, Sapporo-shi, Hokkaido 060-8556 (JP); ITOH, Fumio, Kawasaki-shi, Kanagawa 213-0011 (JP); TOKINO, Takashi, Sapporo-shi, Hokkaido 060-8556 (JP)
(74) Representative: Flaccus, Rolf-Dieter
(86) International application number: PCT/JP2007/059953
(87) International publication number: WO 2007/132844

(56) References cited:
- EP-A- 0 655 505
- WO-A-98/33530
- WO-A-2005/056831
- WO-A1-96/21041
- JP-A- 04 371 162
- JP-A- 05 199 984
- JP-A- 2001 046 314
- JP-A- 2004 049 699
- JP-A- 2006 506 089
- MICHAUX I ET AL: "Repetitive endoscopy and continuous alkaline gastric irrigation in a case of arsenic poisoning." JOURNAL OF TOXICOLOGY. CLINICAL TOXICOLOGY 2000, vol. 38, no. 5, 2000, pages 471-476, XP008117711 ISSN: 0731-3810
- ALFONSO R ET AL: "Isolation and identification of mycobacteria in New World primates maintained in captivity." VETERINARY MICROBIOLOGY 5 MAR 2004, vol. 98, no. 3-4, 5 March 2004 (2004-03-05), pages 285-295, XP002564762 ISSN: 0378-1135
- SAITO NAGAHITO ET AL: "Removal of mucus for ultrastructural observation of the surface of human gastric epithelium using pronase." HELICOBACTER APR 2002, vol. 7, no. 2, April 2002 (2002-04), pages 112-115, XP002564763 ISSN: 1083-4389
- CHEN B. ET AL.: 'Molecular analysis of gastric washings in the diagnosis and monitoring of gastric lymphomas' HUM. PATHOL. vol. 35, no. 5, 2004, pages 582 - 586, XP003019315
- SANUKI M. ET AL.: 'I Naishikyo Kensa Mae Shochizai KPD (Pronase) no Ippan Rinshoteki Kento' GEKKAN CLINICAL REPORT vol. 25, no. 5, 20 March 1991, pages 236 - 241, XP003019316

### SEQUENCE LISTING

<110> (1) Sapporo Medical University
   (2) St. Marianna University, school of Medicine
<120> Method for detecting disease related marker using gastric mucosal
   washing liquid
<130> 2260SI
<140> EP 07743388.6
   <141> 2007-05-15
<150> JP 2006-134878
   <151> 2006-05-15
<160> 5
<170> PatentIn version 3.1
<210> 1
   <211> 25
   <212> DNA
   <213> Artificial sequence
<220>
   <223> PRMD5 sense primer
<400> 1
   tagcgtttag gttcgcgttt ttcgc 25
<210> 2
   <211> 24
   <212> DNA
   <213> Artificial sequence
<220>
   <223> PRDM5 antisense primer
<400> 2
   taccgattcc aaaatccccc gcga 24
<210> 3
   <211> 21
   <212> DNA
   <213> Artificial sequence
<220>
   <223> PRDM5 probe
<400> 3
   tcgggtcgag ttcgattcgg g 21
<210> 4
   <211> 18
   <212> DNA
   <213> Artificial sequence
<220>
   <223> hpu sense primer
<400> 4
   gccaatggta aattagtt 18
<210> 5
   <211> 18
   <212> DNA
   <213> Artificial sequence
<220>
   <223> hpu antisense primer
<400> 5
   ctccttaatt gtttttac 18

## Description

### [Technical field]

The present invention relates to a method for detecting a disease-related marker, more specifically, the method using gastric mucosalwashes,asample containing gastric mucosalwashes, a pretreatment agent used for the method, and an apparatus used for the method.

### [Background art]

Cancer has been the leading cause of death in Japan for the past 20 years. When evaluating the cancer as the cause of death by cancer site, gastric cancer has continuously been among the high-ranked cancers; however, details of gastric-cancer cases have apparently been changing in recent years. While the number of patients with gastric cancer stages III and IV represented by advanced gastric cancer accounted for the majority of patients in the past, currently more than 50% of the patients are those of stage I. Stage I is classified into stage Ia and stage Ib, and both are characterized by the fact that the cancer is limited to the submucosal layer; that is, they are classified as so-called early cancers.
As treatment policy of such early cancers, relatively low-invasive treatment methods are generally adopted, in comparison to extensive regional lymph node resection that has a large influence on the body, and such low-invasive methods include endoscopic local excision, conventional partial gastrectomy, and laparoscopic partial gastrectomy and others. However, different from total gastrectomy that had been the major treatment method until the increase of early gastric-cancer cases, a part of the stomach remains in the above treatment methods, so that care must be taken to the recurrence of cancer in the remaining stomach. Furthermore, since preoperative evaluation of the above early cancers by macroscopic diagnosis does not have a 100% diagnostic performance at present, the total resection ratio does not reach 100%; thus, the judgment and evaluation of remnant cancer has become very important. Under such circumstances, however, regular macroscopic diagnosis using endoscopy, and histological examination of spot biopsy are the only examinations on which we are currently dependent.

In gastric endoscopy, generally, mucosal abnormalities are picked up macroscopically during washing of the stomach. In some cases, surface abnormalities are searched by contrast method using pigment dispersion (indigo carmine solution). Then, when a suspected region is found, a part of the region will be collected as a spot biopsy sample, fixed in formalin, and its histological findings will be evaluated by microscopy.
However, with conventional gastric endoscopy, it is naturally impossible to pick up minute cancer that cannot be macroscopically selected; in addition, spot biopsy can only evaluate tissues by a "spot" and extending the spot into "area" has a physical limitation. Furthermore, regions subjected to endoscopic or laparoscopic treatment are mostly modified by fibrotic response and/or thermocoagulation degeneration, so that their macroscopic evaluation is difficult in most cases. Therefore, when the diagnosis of remnant cancer is made, in many cases the cancer is already in a advanced stage.
Since an increase of the above-described low-invasive treatments is expected, it is urgently required to establish an accurate post-operative diagnostic method adapted to such treatment methods.

Meanwhile, in the diagnosis of digestive disorders, detection of disease-related markers such as nucleic acids and proteins that are specific to a disease of interest has been attempted recently, as a method to complement or replace biopsy with subsequent histological evaluation. For example, non-patent documents 1 and 2 describe that a method to diagnose colon cancer using fecal DNA has demonstrated a certain outcome. Non-patent documents 3, 4 and 5 describe that ascites DNA or serum DNA can be used for the diagnosis of gastric cancer, and non-patent document 6 describes that gastric mucus DNA can be used for the diagnosis of Helicobacter pylori infection, and non-patent document 7 describes that DNA contained in gastric washing liquid from endoscopy can be used for the post-treatment evaluation of lymphoma. However, with the method described in non-patent document 7 for example, a sufficient amount of DNA to perform Southern blotting required for the diagnosis cannot be extracted in approximately half of the samples. Thus, any of the above methods have problems in diagnostic capability and accuracy, and they are still far from practical application.

[Non-patent document 1] Ahlquist DA et al., Gastroenterology. 2000 Nov; 119(5):1219-27
[Non-patent document 2] Osborn NK et al., Gastroenterology. 2005 Jan; 128(1):192-206
[Non-patent document 3] To EM et al., Diagn Mol Pathol. 2003 Jun;12(2):88-95
[Non-patent document 4] Leung WK et al., Br J Cancer. 2005 Jun 20;92(12):2190-4
[Non-patent document 5] Marutsuka T et al., Clin Cancer Res. 2003 Feb;9(2):678-85
[Non-patent document 6] Furuta T et al., J Clin Microbiol. 1996 Oct;34(10):2421-5
[Non-patent document 7] Chen B et al., Hum Pathol. 2004 May;35(5):582-6

MICHAUX I, et al: "Repetitive endoscopy and continuous alkaline gastric irrigation in a case of arsenic poisoning", JOURNAL OF TOXICOLOGY, CLINICAL TOXICOLOGY 2000, vol. 38, no. 5, pp. 471-476, describes a gastrointestinal decontamination treatment of a patient who had ingested trivalent arsenic powder. The treatment included gastric lavage with normal saline, followed by large volume irrigation of the stomach with bicarbonate solution to reduce protracted arsenic absorption.

JP 2001 046314 A relates to an endoscope which comprises a tube for transferring fluid into the endoscope.

JP 2004 049699 A discloses a washing adapter for an endoscope for connecting the endoscope to a washing system which can supply a washing liquid into a duct formed in the endoscope.

WO 2005/056831 A1 relates to PCR detection of pathogenic mycobacteria in clinical specimens such as gastric lavage. After obtaining the clinical specimens from patients, the specimens are clarified from contaminants including mucus by conventional methods, followed by DNA extraction and PCR amplification.

ALFONSO R et al: "Isolation and identification of mycobacteria in New World primates maintained in captivity", VETERINARY MICROBIOLOGY, 5 March 2004, vol. 98, no. 3-4, pp. 285-295, describes a diagnostic method in which clinical samples are obtained by gastric lavage using phosphate-buffered saline, and are then processed by DNA extraction and PCR amplification.

WO 98/33530 A1 discloses a method for detecting abnormal epithelial cell shedding, e. g. of the gastric glands. The method includes in vivo labeling of epithelial cells, e. g. by orally administering a dye-containing labeling composition to a target site. To increase the adhesion of certain dye compounds to the epithelial mucosa, a mucus-removing procedure may be employed prior to administering the labeling composition to the target epithelium.

SAITO NAGAHITO et al: "Removal of mucus for ultrastructural observation of the surface of human gastric epithelium using pronase", HELICOBACTER vol. 7, no. 2, April 2002, pp. 112-115, discloses a method for the removal of mucus from biopsied specimens obtained from patients. The specimens are fixed in glutaraldehyde and then treated with different concentrations of pronase. Due to this mucus removal treatment, ultrastructural observation of the gastric epithelium is improved.

EP 0 655 505 A relates to a process for lysing mycobacteria in an aqueous solution containing a chelating agent, followed by PCR amplification.

CHEN B et al. "Molecular analysis of gastric washings in the diagnosis and monitoring of gastric lymphomas", HUM. PATHOL. vol. 35, no. 5, 2004, pp. 582-586, relates to a diagnostic method in which gastric washing fluid obtained from patients is used for DNA extraction and subsequent PCR analysis and Southern blot analyses.

JP 2006 506089 A relates to methods and system for tissue cell and/or nucleic acid molecule isolation. The method and system utilizes hydrodynamic shear force to disrupt the tissue sample and treating the tissue sample with at least one enzyme for tissue dissociation, e. g. pronase.

SANUKI M et al: "I Naishikyo Kensa Mae Shochizai LPD (Pronase) no Ippan Rinshoteki Kento" GEKKAN CLINICAL REPORT vol. 25, no. 5, 20 March 1991, pp. 236-241, relates to a general clinical study of KPD (Pronase) as a premedication for endoscopy. The results demonstrated that pronase had a superior effect in the removal of mucus from gastric mucosa without any sign of side effects.

### [Disclosure of the invention]

### [Problem to be solved by the invention]

Under these circumstances, the aim of the present invention is to provide a novel method for detecting disease-related markers that does not have the above-mentioned disadvantages.

### [Means of solving the problem]

The inventors of the present invention devoted themselves to the research to solve the above problem, and found the following : by performing a gastric mucus removal treatment prior to the collection of a sample from the stomach of a subject, the mucosal lamellar cells at the location deeper than the mucosal layer, which had previously been difficult to collect, can be reliably collected, and a pH environment wherein damage to nucleic acids is suppressed to a minimal degree can be created, and also a gastric mucosal washes obtained by directly washing the gastric mucosal surface after suction-removal of gastric mucus has extremely excellent characteristics as a sample used for the detection of nucleic acids, and, by analyzing nucleic acids extracted from this washing liquid, disease-related genes can be detected with high frequency; thus, the inventors have accomplished the present invention.

Accordingly, the present invention relates to a method for detecting a disease-related marker selected from the group consisting of cancer-related gene, *H. pylori* and its related genes, EBV and its related genes, and CMV and its related genes; said method comprising a process of extracting a nucleic acid from the sample containing a gastric mucosal wash obtained by washing the gastric mucosa exposed due to an administration of a gastric mucus removal agent and subsequent washing and removal of gastric mucus. The invention also relates to the aforementioned method, wherein the method further comprises a process of adjusting the pH of the sample to between 3 and 11.
The present invention further relates to a method for evaluating therapeutic efficacy of a drug and/or a treatment method, comprising a process of detecting a disease-related marker using the above-described method.
The present invention futher relates to the use of a sample containing a gastric mucosal wash in the above-described method, wherein the gastric mucosal wash is obtained by washing the gastric mucosa exposed due to an administration of a gastric mucus removal agent and subsequent washing and removal of gastric mucus. The invention also relates to the use of said sample, wherein the sample contains 5 µg or more of a nucleic acid.
The invention also relates to the aforementioned use wherein the sample further contains a chelating agent and/or a pH adjuster. The invention also relates to the aforementioned use wherein the pH of the sample is from 3 to 11.
The present invention futher relates to the use of a pretreatment agent containing a gastric mucus removal agent and a pH adjuster that can adjust the pH of a sample to between 3-11, in the above described method.
In addition, the invention relates to the use of a sample collection container for collecting a gastric mucosal washes, wherein the container contains a chelating agent and/or a pH adjuster and is connectable to a suction line of an endoscopic apparatus, in the above-described method for detecting a disease-related marker.

### [Effects of the invention]

Regarding the samples and methods of the present invention, a gastric mucosal washes obtained by washing the gastric mucosal surface, after removal of the mucus attached to the gastric mucosa of a subject, is used, and this is essentially different from the method described in non-patent document 7, wherein a mere gastric washing resulting from endoscopy which contains a large quantity of gastric juice is used. When a mere gastric washing was used as a sample, sufficient DNA for PCR could not be obtained in approximately 20% of the cases, and execution of Southern blotting was impossible in approximately 40% of the cases, as described in non-patent document 7; because certain degrees of physical and economical burdens are required for subjects in the execution of endoscopy, the use of the conventional method, wherein gastric washings that frequently require sample re-collection are used, could never be used in clinical sites. However, when the present method using a gastric mucosal washes is used, a sample containing markers of superior quality can easily be obtained; because a waste fluid derived from endoscopy can be utilized as a sample material, following various advantages are obtained.

Namely, because washing liquids conventionally disposed of are used as materials in the samples and methods of the present invention, various possible risks occurring in other test methods utilizing an endoscope (such as risk of bleeding (biopsy), allergy, development of kidney damages (pigment dispersion method), prolongation of the time of examinations (magnification endoscopy), expensive apparatus (magnification endoscopy, NBI: Narrow Band Imaging) do not newly occur, and in addition, because it is easy to collect samples, almost no burden is applied to physicians operating endoscopy and assisting nurses. Moreover, in the present method, since a sample collection tube is fixed at the middle of the closed circuit of an endoscopic apparatus, samples can be protected from contamination and no additional investment in the facility is required because conventional endoscopic apparatus can be used as it is.

In addition, by using the present method, the evaluation at the "area" level becomes possible only by washing entire suspected mucosal surface. Moreover, since the tissue is not damaged as in the case of biopsy, a test can be performed in subjects in whom hemostatic function or wound healing function deteriorates, or in subjects who take a drug that affects hemostasis or wound healing, such as antiplatelet agents and anticoagulants. Since many of the subjects who require endoscopy are elderly persons, and many of them often regularly use such drugs, this advantage is very important. Furthermore, the present method enables DNA search for genetic or epigenetic abnormalities and simultaneous evaluation of H. Pylori or EB virus, etc. which could be a risk factor of gastric cancer. In particular, regarding the identification of H. Pylori, evaluation of the entire stomach is possible instead of diagnosing regions with low possibilities of bacterial presence such as regions with mucosal atrophy and cancer lesions. When merely the gastric mucus is collected and tested as in conventional cases, it is highly possible to detect markers from dead H. Pylori, resulting in a high rate of false positive; thus the conventional method is absolutely not suitable as a means of judgment in bacterial eradication treatments. However, since the gastric mucus is removed in the present method, the method is superior in picking up viable bacterium invaginating into cells on the mucosal surface.
In addition, using a sample collected by the method of the invention, it is possible to examine sensitivities of various drugs comprising anti-cancer agents.
Furthermore, using samples and methods of the invention, it is possible to determine presence/absence of a disease based on the amount of a nucleic acid contained in a sample, for example presence/absence of gastric tumors; therefore, convenient and inexpensive screening of subjects having diseases becomes possible.

### [Brief description of drawings]

[Fig. 1] Figure 1 illustrates a structure of the sample collection container.
[Fig. 2] Figure 2 is an electrophoretogram showing states of DNA in various samples.

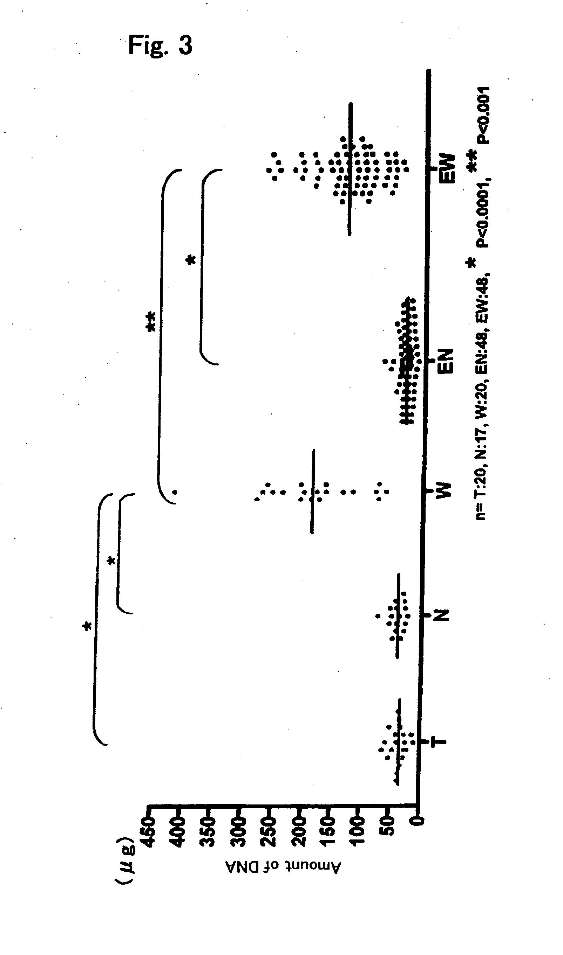

[Fig. 3] Figure 3 is a diagram evaluating amounts of DNA in various samples by spectrophotometry. The axis of abscissa shows kind of samples, and the axis of ordinate shows amount of DNA (µg). The symbols of the axis of abscissa represent the following. T: Biopsy sample of tumor lesion from cancer subject, N: biopsy sample of normal site from cancer subject, W: gastric mucosal washes from cancer subject, EN: biopsy sample from subject diagnosed as normal by endoscopy, EW: gastric mucosal washes from subject diagnosed as normal by endoscopy. The symbol * indicates a significant difference between two groups with P<0.0001 by t-test, ** the same with p<0.001. The horizontal lines in the figure show average values.
[Fig. 4] Figure 4 is a diagram evaluating methylated DNA in various samples (amplification plot).
[Fig. 5] Figure 5 shows diagrams evaluating methylated DNA in various samples (multicomponent plot).
[Fig. 6] Figure 6 is an electrophoretogram showing presence/absence of hpu genes in gastric mucosal washes samples and in biopsy samples. The symbols T and W above the diagram represent a tumor biopsy sample and a gastric mucosal washes sample, respectively, and the numbers after the symbols represent subject numbers described in Example 5.
[Fig. 7] Figure 7 is a graph showing amounts of DNA in samples incubated for 3 h at different pH conditions.
[Fig. 8] Figure 8 is a graph showing amounts of DNA in samples incubated for 24 h at different pH conditions.
[Fig. 9] Figure 9 is an electrophoretogram showing states of DNA in samples incubated for 3 h or 24 h at different pH conditions.
[Fig. 10] Figure 10 shows diagrams evaluating methylated DNA in samples incubated for 3 h or 24 h at different pH conditions (multicomponent plot).

### [Description of symbols]

1: Sample collection container
2: Upper part of the sample collection container
3: Lower part of the sample collection container
4: Endoscope-side connecting portion
5: Aspirator-side connecting portion

### [Best mode for carrying out the invention]

In the following, suitable embodiments of the present invention will be described in detail.
The present invention relates to a sample for testing disease-related makers, which contains a gastric mucosal washes collected from a subject who has received a gastric mucus removal treatment.
In the present invention, "subject" means any individual organism having a stomach, and is preferably animals, more preferably mammals, for example, humans, nonhuman primates, companion animals such as dogs, cats, industrial animals such as cattle, horses, goats, sheep, pigs, and particularly preferably humans. In the present invention, a subject may be healthy, or affected by a certain disease, and may be either during treatment of a disease or after treatment.

In the present invention, a "gastric mucus removal treatment" encompasses any treatment that intentionally removes gastric mucus generally attached to the gastric mucosa, and it typically includes administration of a gastric mucus removal agent to a subject; it also includes a purely physical method wherein water or a physiological saline solution is introduced in the stomach of a subject via an endoscopic apparatus, etc., then the mucus which physically detaches is aspirated. A gastric mucus removal treatment which can minimize the region covered by gastric mucus is preferred, so that it includes, for example, but not limited to, the following treatments: a treatment wherein an agent for dissolving and removing mucus is administered to a subject, and the agent is dispersed in the entire stomach by changing the body position of the subj ect, or, in more simple cases, a treatment wherein an agent for dissolving and removing mucus dissolved in a fairly large amount of a solvent (for example, dissolving 20,000 units of Pronase® MS, 1 g of sodium hydrogen carbonate and 4 ml of Gascon® drop (containing 80 mg of dimethicone) in 100 ml or more of tap water) is administered to a subject, then the subject maintains a seated position at rest for a predetermined time, e.g., 10-15 min.

As a gastric mucus removal agent used for gastric mucus removal treatment, any agent having a characteristic to facilitate washing and removal of gastric mucus may be used, including substances which decompose viscosity-inducing materials in the gastric mucus, such as proteins, saccharides, and glycoproteins. In the present invention, from the viewpoints of high removal rate of gastric mucus and safety of subjects, a proteinase formulation Pronase® is preferable. When Pronase® is used for an adult, typically 20,000 units of Pronase® is dissolved in approximately 50-80 ml of water, and administered orally. To increase its enzymatic activity, preferably it is co-administered with sodium hydrogen carbonate. A typical dose of sodium hydrogen carbonate is 1 g per 1 adult. To increase the efficiency of suction of a gastric mucosal washes, it is also preferable to co-administer an antifoaming agent such as dimethicone (dimethylpolysiloxane) . A typical dose of dimethicone as an antifoaming agent is 40-80 mg per 1 adult.

A gastric mucus removal treatment can be performed at any time point before collection of a gastric mucosal washes; because gastric mucus is continuously produced, preferably the treatment is performed immediately before the collection. However, when a gastric mucus removal agent is used, since it is necessary to ensure a certain time for the agent to react with the gastric mucus, preferably the treatment is performed within 1 h before the collection, and more preferably within 30 min, and furthermore preferably within 20 min. The gastric mucus removal agent can be administered via any route that enables exhibition of desirable effects such as the gastric mucus removal action, and the examples include oral administration, administration via a water supply function of an endoscopic apparatus,a gastric catheter (including oral and transnasal catheters) or a gastric fistula, etc.

In the present invention, a subject may be those who have further received a gastric-acid secretion inhibition treatment. Here, the gastric-acid secretion inhibition treatment includes administration of a gastric-acid secretion inhibitor such as a proton pump inhibitor (PPI) and an H₂ receptor antagonist, etc., before the collection of gastric mucosal washes, for example, from approximately 1 week before the collection. In the present invention, PPI which is superior in maintaining its effect to increase gastric pH (pH holding) is preferred. Examples of the PPI include omeprazole, lansoprazole, and rabeprazole, etc., and examples of the H₂ receptor antagonist include cimetidine, famotidine, ranitidine and roxatidine, etc., but they are not limited thereto. A gastric-acid secretion inhibitor is administered with a general therapeutic dose. For example, in the case of omeprazole, it is 20 mg/day per an adult. The administration route of the gastric-acid secretion inhibitor is not limited particularly, and it can be appropriately selected from oral administration of internal formulations, parenteral administration of injection formulations, and others.

In the present invention, a "gastric mucosal washes" is a liquid that is obtained by washing the gastric mucosa exposed due to a gastric mucus removal treatment, using water flow of a washing liquid. Accordingly, it differs from so-called "gastric washing" obtained by merely washing the gastric mucosa to which gastric mucus still attaches without performing a gastric mucus removal treatment.
In the present invention, preferably the gastric mucosal washes has a sufficient amount of a nucleic acid to perform analysis . Here, the sufficient amount to perform analysis means that the amount of a nucleic acid in the liquid is preferably 5 µg or more, more preferably 10 µg or more, furthermore preferably 50 µg or more, and most preferably 100 µg or more. The amount of a nucleic acid in a gastric mucosal washes may be measured by known methods such as spectrometry. In addition, to ensure high-quality nucleic acids for analysis, preferably the gastric mucosal washes contains substantially no gastric mucus. Here, "containing substantially no gastric mucus" means that the gastric mucosal washes is collected under the following conditions: with the endoscopic and macroscopic observation, the gastric mucus is attached to the gastric mucosa of a subject with 1 point/1 visual field or less, preferably 1 point/2 visual fields or less, more preferably 1 point/3 visual fields or less, and most preferably no point in any visual field.

The gastric mucosal washes can be collected by any known method using washing/suction functions of an endoscopic apparatus or gastric catheters. Collection by endoscopic apparatus is preferable because suction can be performed while monitoring internal conditions of the stomach by its image. Here, an "endoscopic apparatus" refers to any commercially-available endoscopic apparatus; those at least equipped with a scope having at least one water-supply channel and a suction channel, a processor to manipulate and drive the scope, and an aspirator is preferred. Examples of such endoscopic apparatus include, for example, EVIS 200 series, EVIS 240 series, and LUCERA series from Olympus Corporation, FTS 200 system, FTS 400 system from Fujinon Toshiba ES Systems, Co., Ltd., and endoscopic systems from PENTAX Corporation.

Upon collection of a gastricmucosal washes, it is preferable to discard an early suction liquid that contains fairly large amounts of gastric juice and gastric mucus removed from the gastric mucosa, then collect the suction liquid having sufficiently small contents of such ingredients, as a gastric mucosal washes. Here, the "suction liquid having sufficiently small contents" means a suction liquid after the stomach is washed such that the gastric mucus attaches to the gastric mucosa of a subject with 1 point/1 visual field or less, preferably 1 point/2 visual fields or less, more preferably 1 point/3 visual fields or less, and most preferably no point in any visual field, by endoscopic and macroscopic observation.

A sample of the present invention may further contain a chelating agent and/or a pH adjuster to stabilize nucleic acids. The chelating agent and/or pH adjuster may be administered to a subject prior to the collection of a gastric mucosal washes, such as at the time of a gastric mucus removal treatment, or may be added to the gastric mucosal washes after it has been collected in a container, or may be previously added into a collection container. In cases when the chelating agent and/or pH adjuster is previously added to the collection container, it may be present in the form of solid (powder, granule, tablet, capsule, etc.) or liquid (solution, suspension, emulsion, etc.) in the container, or may be attached to the wall surface of the container. Alternatively, the chelating agent and/or pH adjuster may be attached to a part inside the container that does not in contact with the suction liquid during the manipulation of collection, such as on the back of the container top, then after the collection, it may be in contact with the suction liquid by turning the container upside down and mixing. Moreover, when the upper part of the sample collection container that is connected to an endoscopic apparatus and the lower part of the container that receives a gastric mucosal washes are separable, a separate cap is prepared, which is attachable to the lower part of the container and has a chelating agent and/or pH adjuster in its inside surface, then after the sample has been collected, only the lower part is removed from the endoscopic apparatus and said cap is attached, and by turning the container with the cap upside down and mixing the content, the chelating agent and/or pH adjuster provided to the cap can be introduced into the collected gastric mucosal washes.
A temperature upon addition of the agent is not particularly limited so long as it is within a range that disease-related markers in a sample are not degenerated; from the viewpoint of the protection of markers, a temperature of refrigeration (around 4°C) is preferable.

Any known chelating agents may be used, which include various chelating agents such as ethylenediaminetetraacetate (EDTA) (such as EDTA4H, EDTA2Na-2H₂O, EDTA3Na-2H₂O, EDTA3Na-3H₂O, EDTA4Na·4H₂O, EDTA4Na·liquid), hydroxyethylethylenediaminetriacetate (HEDTA) (such as HEDTA3Na·3H₂O, HEDTA3Na·liquid), dihydroxyethylethylenediamine (DHEDDA) (such as DHEDDA2Na), 1,3-propanediaminetetraacetate (1,3-PDTA) (such as 1,3-PDTA4H), diethylenetriaminepentaacetate (DTPA) (such as DTPA5H, DTPA5Na, DTPA-FeAM), triethylenetetraminehexaacetate (TTHA) (such as TTHA6Na), nitrilotriacetate (NTA) (such as NTA3Na·H₂O), gluconate (such as gluconate Na), hydroxyethyliminodiacetate (HIMDA) (such as HIMDA2Na), L-aspartate-N,N-diacetate (ASDA) (such as ASDA4Na), aminotrimethylenephosphonate (NTMP) (such as NTMP), hydroxyethanephosphonate (HEDP) (such as HDEP); EDTA mixtures, EDTA derivatives, EDTA metal salts, and nonaqueous chelating agents. From the viewpoint that the chelating agent is necessary as a chelating reagent which adjusts the pH from neutral to slight alkaline and removes divalent metal ions which activate DNase, an EDTA chelating agent is preferred. The amount of a chelating agent is not particularly limited; in the case of 0.5 M EDTA with pH 8, it is preferably 0.1-5.0 ml per 50 ml of a collected liquid, more preferably 0.2-2.0 ml, and particularly preferably 0.2-1.0 ml.

Regarding the pH adjuster, any known pH adjusters may be used; those which can avoid the damage of DNA under an acidic environment of pH 7 or less or under an extreme alkaline environment are preferable, and examples include sodium hydrogen carbonate, NaOH and HCl. A suitable amount of addition of a pH adjuster is those that can adjust the pH of a sample to preferably between 3-11, more preferably between 5-11, and particularly preferably between 7-11, and most preferably between 9-11. For example, in the case of sodium hydrogen carbonate, it is preferably between 0.5-2.0 g per 50-ml sample, more preferably between 0.7-1.5 g, and particularly preferably 1.0 g. In addition, in the case when a pH adjuster is administered to an adult subject prior to collection, it is preferably between 0.5-2.0 g, more preferably between 0.7-1.5 g, and particularly preferably 1.0 g.

A collection method of gastric mucosal washes is not particularly limited; when an endoscopic apparatus is used, a method wherein at least one sealable sample collection container is connected to either a manipulation part in a suction line, a connector part, a suction tank, an aspirator, or any portion between these, may be adopted. Considering the ease of washing and sterilization as well as the risk of contamination, the sample collection container is preferably connected between the connecter part and the suction tank. In addition, from the viewpoints of operability and ease of washing, preferably, the sample collection container is detachably connected. Typically, for example, the suction tube connecting the connector to the suction tank is detached from the suction tank and connected to the connecting portion of the inflow side of the sample collection container, and the connecting portion of the outflow side of the sample collection container is connected to the suction tank. An endoscopic apparatus is preferably equipped with a member to hold the sample collection container. Regarding the above sample collection container, only one container may be connected, or a plurality of containers may be connected in series or in parallel.

The disease-related markers of the present invention include any markers known in this technical field. Examples include a nucleic acid, the presence and the amount of which correlate with a specific disease. Such markers can preferably be present in a gastric mucosal washes, and they include, for example, cancer-related genes such as APC, K-RAS, H-RAS, N-RAS, p53, P16, CHFR, RASSF family, SFRP family, MINT family, MGMT, RUNX family, SMAD family and PRDM family, as well as H pylori and its related genes (CagA, hpu, cagPAI, BabA, AlpA/B, HopZ, iceA, VacA, etc.), EBV and its related genes, CMV and its related genes and others, but they are not limited thereto, and various markers which have not yet been known at present but will be discovered in the future may also be included.
Furthermore, as a disease-related marker of the present invention, the amount itself of a nucleic acidmaybe used. Namely, in the present method, an amount of DNA and/or an amount of RNA in a sample can be utilized as an index to determine the presence/absence of a disease, such as a tumor existing in the stomach, in particular gastric cancer. In concrete terms, when the presence/absence of a tumor in the stomach is to be determined by the amount of DNA in a sample, it is determined as follows, for example: if the amount of DNA in the sample of the present invention is equal to or greater than a predetermined amount, or if it is greater than the amount of DNA in the sample of a subject without cancer by a predetermined ratio, the subject from which the sample of the present invention was obtained can be judged to have a high possibility of having gastric cancer. Concrete values of such predetermined amount or predetermined ratio (cut-off values) can be set appropriately by performing subject-control studies for a given sample-preparation and detection protocols.

The present invention also relates to a pretreatment agent for the collection of gastric mucosal washes, comprising a gastric mucus removal agent. As the gastric mucus removal agent contained in said pretreatment agent, any of the above-mentioned agents may be used. Said pretreatment agent may further comprise one or more agents selected from the group consisting of pH adjusters, anti-foaming agents and chelating agents. Specific examples and dosage of such ingredients are as described above. Said pretreatment agent may be in any dosage form suitable for oral administration, such as powder, granule, tablet, capsule, solution, suspension, emulsion, gels, and syrup. Here, various additives suitable for each dosage form, such as excipients, disintegrants, binders, lubricants, coating agents and solvents may also be contained (refer to, for example, Hyojun Yakuzaigaku (Standard Pharmaceutics), Eds. Yoshiteru Watanabe, et al., Nankodo, 2003). In said pretreatment agent, each of the above-mentioned ingredients may be contained in a single formulation, or may be contained in two or more separate formulations. In the latter case, each formulation may be administered separately, or administered simultaneously after mixing. Since it is advantageous that said agent rapidly disperses in the entire stomach, a dosage form that results in a liquid state upon administration, such as solution, suspension, emulsion and syrup is preferable. In addition, solid dosage forms such as powder, granule, tablet and capsule are also preferable because they can be dissolved or suspended into a liquid such as water prior to administration so that they can achieve an effect similar to that of liquid dosage forms.

The pretreatment agent of the present invention contains each ingredient with an amount that can exhibit desired effects such as removal of gastric mucus or stabilization of nucleic acid, preferably by a single dose. Typically, the amount for an adult is 20,000 units of Pronase®, 1 g of sodium hydrogen carbonate, and 40-80 mg of dimethicone. The amount of each ingredient may be appropriately adjusted with consideration given to subject's general health conditions, age, body weight, state of gastric mucosa, amount of gastric mucus, timing of administration, concomitantly-used drugs, and history of treatment.
The pretreatment agent may be administered via any route that enables exhibition of desirable effects such as gastric-mucus removal action, pH-adjustment action, and chelating action, and examples of such route include oral administration, administration via a water-supply function of an endoscopic apparatus, a gastric catheter (including oral and transnasal catheters) or a gastric fistula, etc.

The present invention also relates to a method for detecting disease-related markers, comprising a process for extracting nucleic acids from a sample of the invention.
In the present method, the extraction of disease-related markers from samples may be performed using any known methods. Typically, nucleic acids are extracted from a sample as follows: the sample is subjected to centrifugal separation, and the obtained pellet is re-suspended in an appropriate medium such as PBS or physiological saline solution, then digested by a proteinase such as proteinase K, from which proteins are removed by an organic solvent such as phenol and chloroform, and nucleic acids are precipitated by ethanol, etc. Concrete protocols are not described in detail here, because they are described in various references regarding genetic engineering (for example, Chomczynski,P., Sacchi, N.:Anal. Biochem., 162: 156-159, 1987; Masami Muramatsu and Masashi Yamamoto, eds., Shin Idenshikogaku Handobukku (New Genetic Engineering Handbook), 4th revised version, Yodosha, Oct. 2003, pp.20-29).
The sample is preferably subjected to an extraction treatment immediately after collection, but can be stored for a certain time before extraction, for example, approximately 12 h, or approximately 24 h. Preferable temperature for storage is, from the viewpoint of protection of disease-related markers, preferably between -80°C and 20°C, more preferably between -80°C and 10°C, particularly preferably between -80°C and 4°C. In cases when samples are stored, they are preferably stored, after centrifugation, resuspended in an appropriate medium mentioned above.

The extracted nucleic acids can then be detected by various detection methods suitable for target disease-related markers, including various nucleic-acid amplifi cat ion methods such as PCR, nucleic acid sequence-based amplification (NASBA), transcription mediated amplification (TMA), ligase chain reaction (LCR), strand displacement amplification (SDA), loop-mediated isothermal amplification (LAMP), isothermal and chimeric primer-initiated amplification of nucleic acids (ICAN) and branched DNA, as well as Southern blotting, Northern blotting, RNase protection assay, microarray method, dot blot or slot blot method and others.
In particular, when the marker is an epigenetic methylated DNA, methods such as bisulfite sequencing, methylation-specific PCR (MS-PCR), combined bisulfite restriction assay (COBRA), MS-SNuPE, bisulfite-SSCP, differential methylation hybridization (DMH), MethyLight assay, and pyrosequencing may be used for the detection.
In the quantification of nucleic acids, any known methods may be used, including spectroscopy wherein absorbance at the absorption maximum wavelength of around 260 nm is measured, and methods using various reagents which stain nucleic acids such as ethidium bromide, 4,6-diamine-2-phenylindole (DAPI), acridine orange, Mupid®-STAIN eye (Advance Co., Ltd.), diphenylamine reagent, Hoechst 33258 (H33258), Quant-iT PicoGreen dsDNA Reagent (Invitrogen), Quant-iT RiboGreen® RNA Reagent (Invitrogen), Gel Indicator RNA Staining Solution (Funakoshi Co., Ltd.), SYBR® Green I or II, SYBR® Gold, GelRed, etc.

Detection results of a test sample of a subject is compared with those of a predetermined control sample, for example, a sample collected from the same subject but in a period when the subject had apparently been known not to have the target disease or the subject had the target disease but its stage of progress was known to be earlier than at present, or a sample obtained similarly from other subj ects who are apparently known not to have the target disease; then, if the marker level of the target disease is increasing with presence or progression of the target disease, and when the marker level in the test sample is higher than that in the control sample, this marker level indicates the presence or progression of the target disease, and when it is lower, it indicates the absence or regression of the target disease. Similarly, if the marker level of the target disease is decreasing with presence or progression of the target disease, and when the marker level in the test sample is lower than that in the control sample, this marker level indicates the presence or progression of the target disease, and when it is higher, it indicates the absence or regression of the target disease. Thus, the method of the present invention can be used for the diagnosis of diseases, and such diagnostic method is also encompassed in the present invention.

Furthermore, using the method of the present invention, it is possible to evaluate therapeutic efficacy of a specific drug or a treatment method. For instance, by comparing marker levels prior to the treatment and those after the treatment, or by comparing marker levels at a certain time point during the treatment and those at a later different time point during the treatment, if this comparison shows that the progression of the target disease is slowed or terminated, or that the target disease is eliminated, then the therapeutic efficacy is verified; if the comparison shows that no influence is made on the progression of the target disease, then no therapeutic efficacy is verified. In particular, when the disease is a cancer and the treatment method is a removal surgery, since presence/absence of remnant cancer can be confirmed easily, this embodiment is extremely useful.
The method of the present invention can be used for monitoring presence/absence of recurrence of diseases after completion of treatments. In this case, samples are regularly collected from a patient in whom treatment has been completed, and presence/absence of the disease is examined using the same method as above based on marker levels.

The present invention relates to a sample collection container for the collection of gastric mucosal washes, comprising a chelating agent and/or a pH adjuster, which is connectable to a suction line of an endoscopic apparatus.
The structure of the present container is not particularly limited as long as it can receive a washing liquid from a suction line of an endoscopic apparatus; from the viewpoints such as efficiency of liquid-collection work and risk of contamination, a structure constituting a closed circuit with said suction line is preferable. Examples of such sample collection container include those wherein the upper part of the sample collection container has at least one connecting portion connected to the suction tube or suction channel of the scope side in an airtight manner, and at least one other connecting portion connected to the suction tube or suction channel of the aspirator side in an airtight manner, but they are not limited thereto. In the above embodiment, it is preferable that the upper part of the container has a cap structure, which is detachable from the lower part of the container. The upper part and the lower part of the container are preferably joined in an airtight manner by a screw or a flipper and the like. In addition, preferably at least the lower part of the sample collection container can be mounted directly on a centrifugal machine.

The connecting portion is not particularly limited as long as it is made of a hollow member, but is preferably connectable to a tube provided in the endoscopic apparatus and resistant to gastrointestinal fluid, etc. This member may be hard or flexible, may be attached to or integrated into the upper part of the container. The length of the connecting portion is not also particularly limited; when the connecting portion has a form of a flexible tube, preferably it has a sufficient length to reach the connecting portion of the endoscopic apparatus. Examples of such sample collection container include Argyle Specimen Collection Container (Type O, trap volume: 50 ml, Tyco Healthcare Group LP, Medical device approval number: 16200BZZ00045, Cat No. 2583-50) and others.
The above connecting portions may be equipped with a means for opening/closing such as a cock, valve, flap and three-way cock, and/or a bypassing means that connects the suction line of the scope side with the suction line of the aspirator side by bypassing the sample collection container. Such a means may be integrated into the connecting portion or the upper part of the container, or may be made as a detachable separate member. Furthermore, such a means may be integrated to the endoscopic apparatus or may be provided thereto as a detachable separate member.

The container of the present invention comprises a chelating agent and/or a pH adjuster prior to the collection of a sample. Accordingly, it is not necessary to add these agents after collecting samples. Examples of the chelating agent and/or pH adjuster contained in the container of the present invention and their forms are as described above; an embodiment in which a predetermined amount of such agent is released depending on the water level of the washing liquid inside the sample collection container is preferable. In concrete terms, such examples include a case wherein the above agents are attached to the inner wall of the container, a case wherein the above agents formulated into a rod shape having a length corresponding to the height of the container, or such a rod-shaped medium immersed or coated with the agents, are placed in the container. Alternatively, the present container may have the following structure: the above agents or a reservoir which contains said agents (e. g. , an aqueous pack or capsule) are provided in a part inside the container that is not in contact with the suction liquid during the collection manipulation, such as on the back of the container top, then after the sample collection, the container is, for example, turned upside down to make the agents in contact with the liquid, so that the agents are introduced into the liquid. Alternatively, when the upper part of the sample collection container that is connected to an endoscopic apparatus and the lower part of the container that receives a gastric mucosal washes are separable, a separate cap may be prepared, which is connectable to the lower part of the container and has at its inside surface a chelating agent and/or a pH adjuster themselves or a reservoir comprising such agents, then after the sample has been collected, only the lower part is removed and said cap is attached, and by turning the container with the cap upside down and mixing the content, said agents can be introduced into the collected gastric mucosal washes. In the present invention, from the viewpoints of operability and ease of production, it is preferable that the above agents are attached to the inner wall of the container, or are provided on the inside surface of the cap. In the former case, the above agents can be attached using any known methods such as spray coating.

The volume of the sample collection container is not particularly limited; from the viewpoints of workability and installation space, it is preferably between 10-100 ml, more preferably between 30-50 ml, and particularly preferably 50 ml.
The material of the present container is not particularly limited, and is made of any known materials; polypropylene containers are preferred from the viewpoints of strength and economy.

The present invention also relates to an endoscopic apparatus equipped with the above sample collection container. Examples of the endoscopic apparatus that can be used and the mounting method of the sample collection container to said apparatus are already described above.

### [Examples]

In the following, the present invention is explained in detail based on examples; however, the present invention is not limited to these examples.

### Example 1: Sample collection container

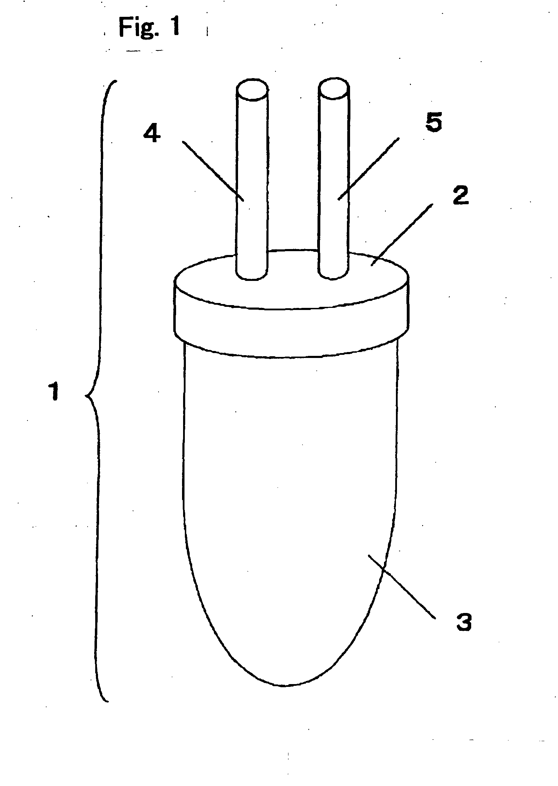

Figure 1 shows an example of the sample collection container of the present invention. The sample collection container (1) consists of the cap-shaped upper part of the sample collection container (2) and the lower part of the sample collection container (3) with a volume of 50 ml, and the two parts are air-tightly joined by screwing. The upper part of the sample collection container (2) is equipped with two connecting portions, i.e., the Endoscope-side connecting portion (4) and the aspirator-side connecting portion (5), enabling airtight connection with a suction line of an endoscopic apparatus. In addition, 0.5 ml of 0. 5-M EDTA (special grade, Wako Pure Chemical Industries, Ltd.) is sealed into the sample collection container (1).

### Example 2: Preparation of samples

From the suction bottle of the endoscopic apparatus (EVIS LUCERA, Olympus Corporation), a suction tube that connects the connector was dismantled and connected to the Endoscope-side connecting portion of said sample collection container, and at the same time, the aspirator-side connecting portion of said sample collection container was connected to the connecting portion of the scope side of the suction bottle, thus forming a closed circuit.
To human subjects with various gastric disorders, the pretreatment agent prepared by dissolving 20, 000 units of Pronase® (Pronase® MS, Kaken Pharmaceutical Co., Ltd.) and 1 g of sodium hydrogen carbonate into a solution of diluting 4 ml of dimethicone (Gascon® drop, Kissei Pharmaceutical Co., Ltd., this contains 80 mg of dimethylpolysiloxane) in 50-100 ml of tap water was administered, then 10-15 min later, the subjects were rotated around their body axis for 2 to 3 times while they were lying, and subjected to normal endoscopy; at this time, the first suction liquid was discarded until the number of attachment of gastric mucus became 1 points/1 visual field by endoscopic and macroscopic observation, then approximately 50 ml of gastric mucosal washes was collected in the sample collection container. After the collection, the sample collection container was removed and stored at 4°C until analysis. The range of pH in all samples was between 7.5-10.0.
For comparison, biopsy samples from lesions and non-lesions of the above subjects, and their serum samples were collected and stored at 4°C.

To extract DNA, each sample was centrifuged at 2700 rpm and 4°C for 15 min, then after removal of the supernatant, the pellet was re-suspended in 4.5 ml of SEDTA. To this suspension, 0.5 ml of 10% SDS and 50 ml of 20-mg/ml proteinase K (Code No. 9033, Takara Bio Inc.) were added respectively, and the resultant was incubated at 55°C for 1 h. Then 5 ml of phenol (UltraPure Buffer-Saturated Phenol, Invitrogen life technologies) was added and the resultant was mixed by turning the container upside down, centrifuged at 2700 rpm and 4°C for 15 min, and the supernatant was transferred to a new tube. This manipulation was repeated for further 1-2 times, and after changing the solvent to chloroform (Wako Pure Chemical Industries, Ltd.) with the same amount, the manipulation was repeated for further 1-2 times. Five ml of glycogen (Cat#9510, Ambion) and 9 ml of 100% ethanol were added, and the resultant was mixed by turning the container upside down, then incubated at 4°C for 12 h. Subsequently, the sample was centrifuged at 2700 rpm and 4°C for 15 min and the supernatant was discarded, the pellet was suspended in 10 ml of 70% ethanol, centrifuged at 2700 rpm and 4°C for 15 min and the supernatant was discarded, the resultant was dissolved in 200 ml of purified water, giving a sample for DNA analysis.

### Example 3: Evaluation of quality and quantity of DNA

### i) Evaluation of quality of DNA

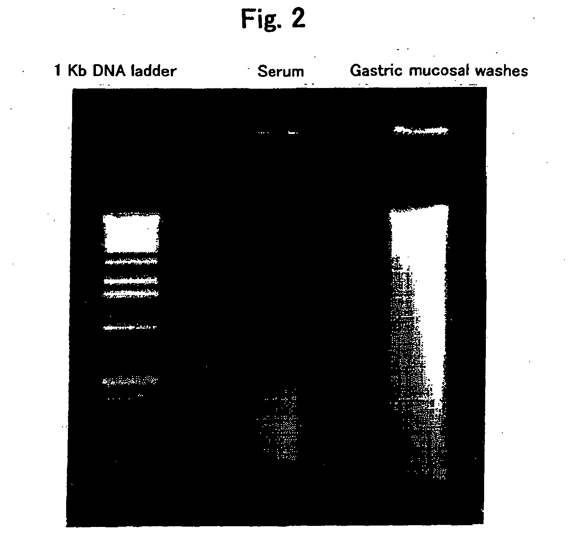

The quality of DNA contained in the above samples derived from human subjects with gastric cancer was evaluated by electrophoresis. Electrophoresis was performed in 1% agarose gel at 100 V for 30 min, by diluting 1-2 µg of a DNA sample to 10 µl of H₂O. Figure 2 shows the evaluation results by electrophoresis. The left lane indicates a positive control (1Kb DNA extension ladder, Cat.No. 10511-012, Invitrogen), and the middle lane indicates a serum sample, and the right lane indicates a gastric mucosal washes. Any of the results demonstrates that the gastric mucosal washes comprises DNA with a quality better than that in the serum samples and comparable to that in the biopsy samples.

### ii) Evaluation of quantity of DNA

The amounts of DNA collected from the samples derived from human subjects with gastric cancer were evaluated by spectrometry. The following samples were used: from subjects with gastric cancer, gastric mucosal washes collected in accordance with the method described in Example 2 (W: approximately 50-100 ml, n=20), biopsy samples from cancer lesions (T: n=20) and biopsy samples from non-lesions (N: n=17) (2 samples with a size of 6 x 6 mm at each location) ; from subjects with gastritis in whom no cancer lesion is endoscopically observed (normal subjects by endoscopic diagnosis), gastric mucosal washes collected similarly (EW: approximately 50-100 ml, n=48), and biopsy samples of gastric mucosa (EN: gastric middle body, 2 samples with a size of 6 x 6 mm, n=48). Regarding spectrometry, a NanoDrop ND-1000 spectrometer (AGC Techno Glass Co. , Ltd.) was used in accordance with the manufacturer's instructions. Namely, measurements were performed by placing 1 µl of a sample on a measurement mount. Figure 3 shows results of the spectroscopic evaluation. Here, statistically significant differences between the groups were evaluated using non-paired Student's t-test (GraphPad PRISM was used).
As is apparent from Fig. 3, in the subjects with gastric cancer, the amounts of DNA contained in the gastric mucosal washes are significantly larger than those in the biopsy samples from cancer lesions or non-lesions (P<0.0001). In the subjects without cancer as well, the amounts of DNA contained in the gastric mucosal washes are significantly larger than those in the biopsy samples (P<0.0001). Moreover, in both of the above gastric mucosal washes, a predetermined band could be obtained by PCR, and methylation assay of DNA by pyrosequencing was possible. Thus, the samples of the present invention were confirmed to be suitable for the detection of nucleic acids both quantitatively and qualitatively.
Furthermore, it was demonstrated that the amounts of DNA contained in the gastric mucosal washes from the subjects with gastric cancer are significantly larger than those from the subjects without cancer (P<0.001). This means that the amount of DNA contained in a gastric mucosal washes can be used as an indicator to determine presence/absence of tumors in the stomach. In contrast, no statistically significant difference was observed for the biopsy samples between subjects with gastric cancer and subjects without cancer. In addition, because a large quantity of DNA can be collected from subjects with gastric cancer, the detection of a specific DNA marker with a higher sensitivity and accuracy becomes possible.

### Example 4: Detection of methylated DNA

Specific methylated DNA in samples derived from human subjects with gastric cancer was evaluated by MethyLight assay that is based on real-time PCR. As a disease-related marker to be measured, PRDM5 was used. This gene is known to be methylated with high frequency in cancer cells (refer to Deng.Q., Haung.S. Oncogene; 23, 4903-4910, 2004). First, DNA samples extracted in Example 2 (regarding biopsy samples, those from 2 locations were used) were treated with sodium bisulfite. Namely, 2 µg of DNA was mixed into 50 µl of H₂O, into which 5.5 ml of 2-M NaOH was added and the resultant was incubated at 37°C for 10 min. Thirty µl of hydroquinone and 520 µl of sodium bisulfite were added to the resultant, then a mineral oil was added to the supernatant, and they were incubated at 50°C for 16 h. Subsequently, the reaction solution was transferred to a new microtube, to which 1000 µl of Wizard® resin (Promega) was added, drainage was performed via a filter, and drainage was performed again with 2 ml of 80% isopropanol. To the resultant solution, 5.5 µl of 3-M NaOH, 1 µl of glycogen (20 mg/ml), 66 µl of 5-M ammonium acetate and 310 µl of 100% ethanol were added, and the resultant was incubated at -80°C for 1 h, centrifuged for 30 min, then 70% ethanol was added to the pellet obtained and they were further centrifuged for 5 min. The obtained pellet was dissolved in 20 µl of H₂O, which was used for PCR.

PCR was performed as follows. First, a reaction mixture (total 50 µl) consisting of 1 µl of a DNA sample after treatment with sodium bisulfite, 25 µl of TaqMan® mix, 1 µl of a primer (sense/antisense), 2 µl of a TaqMan® probe and 21 µl of H₂O was prepared for each sample obtained. As the primer and probe, the followings were used.
Sense primer: TAGCGTTTAGGTTCGCGTTTTTCGC (SEQ ID NO: 1)
Antisense primer: TACCGATTCCAAAATCCCCCGCGA (SEQ ID NO: 2)
Prove: TCGGGTCGAGTTCGATTCGGG-MGB (SEQ ID NO: 3)

Using a real-time PCR system (7900 Fast, Applied Biosystems), the above reaction mixtures were PCR-amplified with the conditions listed in Table 1, and fluorescence was detected.
[Table 1]

**Table 1**

| | | |
|---|---|---|
| 95°C | 20 s | |
| 95°C | 15 s | 50 cycles |
| 60°C | 1 min | |

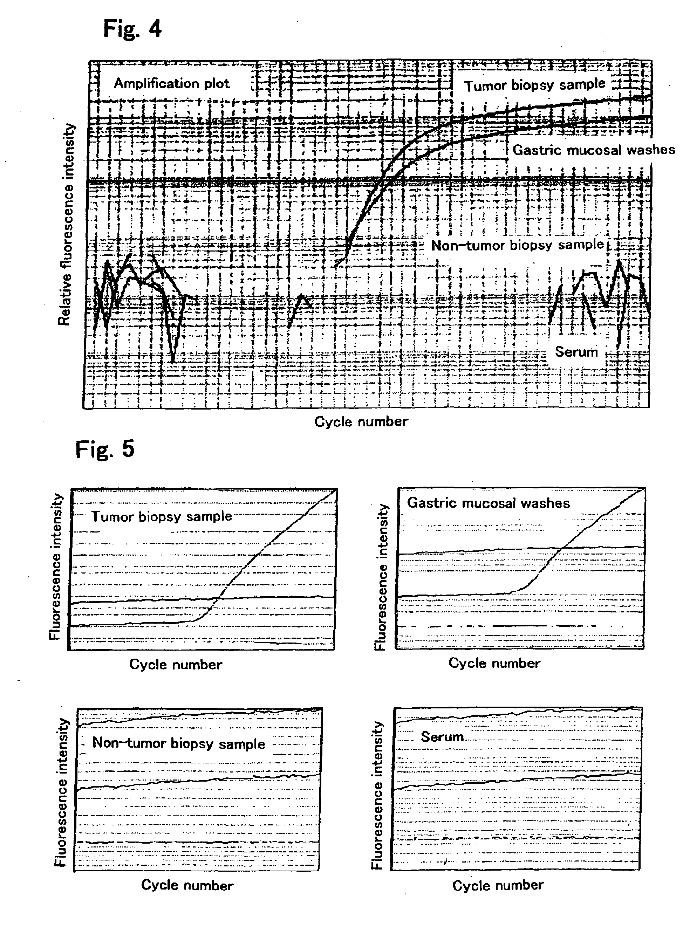

Figures 4 and 5 show the results. These figures demonstrate that methylated DNA cannot be detected from the serum samples, but can be detected from the gastric mucosal washes with sensitivity comparable to that of the biopsy samples.

### Example 5: Detection of hpu (H. pylori urease) gene

The presence of hpu genes in gastric mucosal washes and biopsy samples from 5 human subjects with various gastric disorders was evaluated by PCR. The 5 subjects are as listed in Table 2 below. Here, a rapid urease test (RUT) was performed using Helicocheck® (Otsuka Pharmaceutical Co., Ltd.) in accordance with the manufacturer's instructions. In concrete terms, a substrate reagent solution was prepared by dissolving 20 mg of urea and 4 µg of phenol red into 2.5 ml of a dissolving solution; 5 drops of this reagent solution (approximately 0.2 ml) and an appropriate amount of a biopsy sample were mixed in a reaction cup, left at rest at room temperature for 2 h, then a change in the color of the reagent solution was visually observed.
[Table 2]

**Table 2**

| Subject No. | Kind of disorder | RUT result |
|---|---|---|
| 300 | Gastric cancer (type 4) | - |
| 301 | Gastric cancer (type 3) | + |
| 304 | Gastric cancer (type 4) | + |
| 400 | Gastric ulcer (A2-stage) | + |
| 401 | Gastritis | - |

The hpu gene is one of the markers of pathogenic H. pylori, which is deeply involved in gastric disorders such as gastric cancer and gastric ulcer (refer to, for example, Clayton CL et al. J Clin Microbiol.1992 Jan; 30(1):192-200). PCR was performed as follows. A reaction mixture consisting of 1 µl of a DNA sample, 5 µl of 10xbuffer, 0.85 µl of dNTP, 1 µl of a primer, 0.2 µl of HS-Taq, and 42 µl of H₂O was prepared for each of the above samples, and PCR was performed with the conditions listed in Table 3; amplified products were subjected to electrophoresis with 2.5% agarose gel. As the primer, the followings were used. Sense primer (HPU-1): 5'-GCCAATGGTAAATTAGTT-3' (SEQ ID NO: 4) Antisense primer (HPU-2): 5'-CTCCTTAATTGTTTTTAC-3' (SEQ ID NO: 5)
[Table 3]

**Table 3**

| | | |
|---|---|---|
| 94°C | 5 min | |
| 94°C | 1 min | 35 cycles |
| 45°C | 1 min | |
| 72°C | 5 min | |
| 72°C | 7 min | |

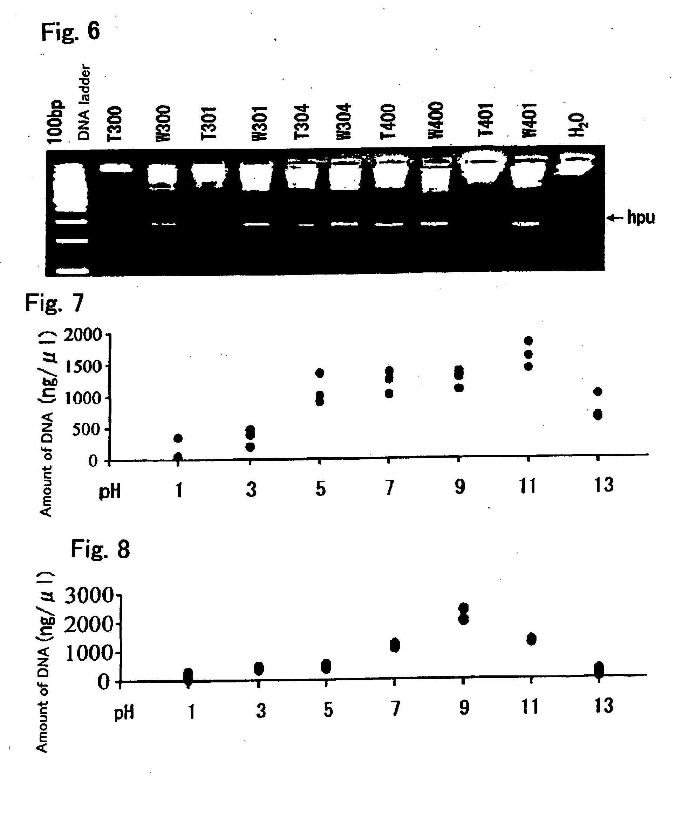

Figure 6 shows the results. This figure demonstrates that hpu genes can be detected from gastric mucosal washes, whereas its detection from biopsy samples has been impossible. Moreover, while 2 out of 5 samples which must be positive were judged to be negative in the biopsy samples by RUT, no such errors occurred in the examination of the gastric mucosal washes. This indicates that gastric mucosal washes exhibit high sensitivity and high specificity with superior diagnostic ability.

### Example 6: Effect of pH in samples on amounts of DNA collected

Using a colon cancer cell strain HCT116, the degree of damage of DNA by different pH values in samples was investigated with an amount of DNA collected as an index. In concrete terms, HCT 116 cells in culture were collected, and exposed for 3 or 24 hr to SEDTA having different pH values of 1, 3, 5, 7, 9, 11 or 13, adjusted using NaOH and HCl. Here, the exposure time of 3 h means the maximum exposure time to gastric juice when DNA is extracted immediately after the collection of the sample, and the exposure time of 24 h means the maximum exposure time to gastric juice when DNA is extracted after the sample collected is transported to a place of examination without any treatment.
After incubation with different pH values for different durations, pH in the sample was re-adjusted to 7 using NaOH and HCl, then DNA was extracted by phenol-chloroform method (refer to Example 2), and the amount of DNA collected was measured by a NanoDrop ND-1000 spectrometer (refer to Example 3). Results are shown in Figs. 7 and 8. We can see that fairly large amounts of DNA can be collected from the samples with pH values between 5-11 after 3-h exposure, and from the samples with pH values between 7-11 after 24-h exposure. In particular, large amounts of DNA could be consistently extracted when the pH values of the samples were between 9-11.

### Example 7: Effect of pH in samples on DNA quality

To investigate the effect of pH in samples on the quality of DNA collected, DNA fragmentation and resistance of DNA modification were examined.

### (1) DNA fragmentation

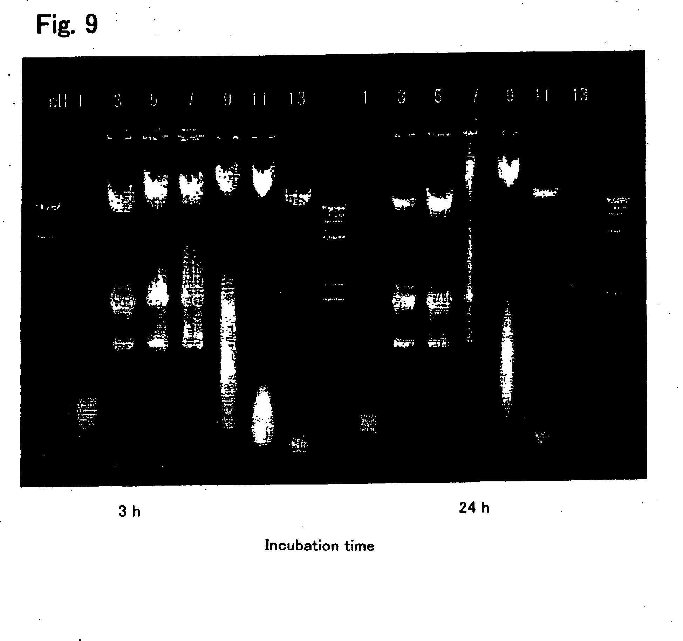

Each of the DNA samples extracted in Example 6 was subjected to electrophoresis with 1% agarose gel (Fig. 9). Regardless of the exposure time, a band could be detected in the samples exposed to the conditions of pH values between 3-11. In addition, it was found that as the condition becomes more basic, fragmentation occurs with lesser frequency.

### (2) Resistance of DNA modification

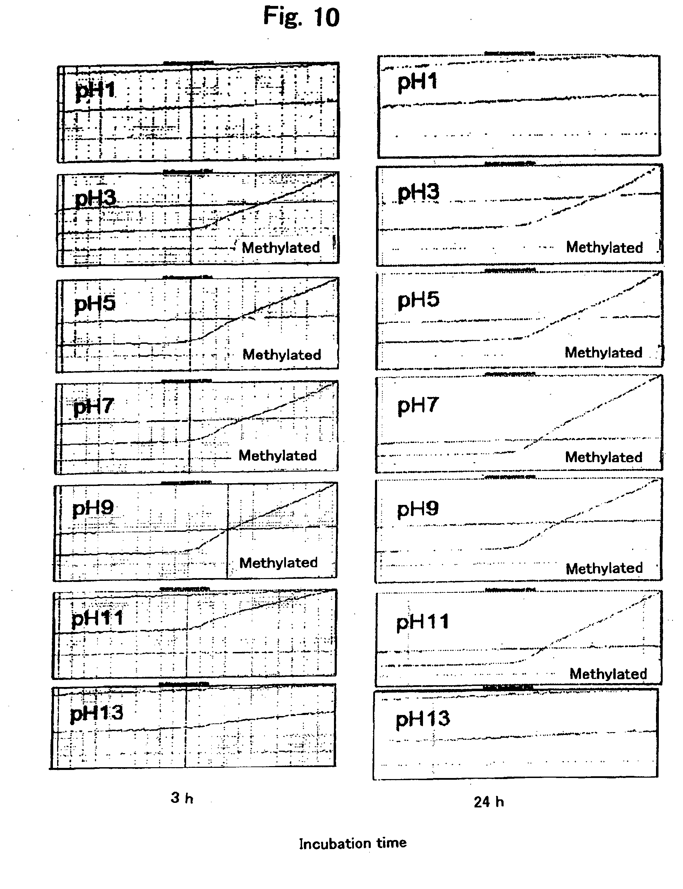

Whether the DNA collected can have a quality to resist a treatment with bisulfite used for the detection of methylated DNA was investigated. In concrete terms, each of the DNA samples extracted in Example 6 was subjected to the treatment with sodium bisulfite and the detection of methylated DNA by MethyLight assay as in Example 4, then samples in which their fluorescence intensity curve is rising were determined to be methylation positive (Fig. 10). Similar to the above results (1), regardless of the exposure time, methylated DNA could be detected in the samples exposed to the conditions of pH values between 3-11. In addition, methylated DNA could be detected with a more consistent manner in the samples exposed to the conditions of pH values between 7-9.

## Claims

1. A method for detecting a disease-related marker selected from the group consisting of cancer-related gene, *H. pylori* and its related genes, EBV and its related genes, and CMV and its related genes; said method comprising a process of extracting a nucleic acid from the sample containing a gastric mucosal wash obtained by washing the gastric mucosa exposed due to an administration of a gastric mucus removal agent and subsequent washing and removal of gastric mucus.

2. The method according to claim 1, further comprising a process of adjusting the pH of the sample to between 3 and 11.

3. A method for evaluating therapeutic efficacy of a drug and/or a treatment method, comprising a process of detecting a disease-related marker using the method according to claim 1 or 2.

4. Use of a sample containing a gastric mucosal wash in the method of any one of claims 1 to 3, wherein the gastric mucosal wash is obtained by washing the gastric mucosa exposed due to an administration of a gastric mucus removal agent and subsequent washing and removal of gastric mucus.

5. The use according to claim 4, wherein the sample contains 5 µg or more of a nucleic acid.

6. The use according to claim 4 or 5, wherein the sample further contains a chelating agent and/or a pH adjuster.

7. The use according to any one of claims 4 to 6, wherein the pH of the sample is from 3 to 11.

8. Use of a pretreatment agent containing a gastric mucus removal agent, and a pH adjuster that can adjust the pH of a sample to between 3-11, in the method of any one of claims 1 to 3.

9. Use of a sample collection container containing a chelating agent and/or a pH adjuster and is connectable to a suction line of an endoscopic apparatus, in the method of any one of claims 1 to 3.

## Patentansprüche

1. Verfahren zum Nachweis eines krankheitsbezogenen Markers, der aus der Gruppe bestehend aus krebsbezogenem Gen, *H*. *pylori* und damit assoziierten Genen, EBV and damit assoziierten Genen, sowie CMV und damit assoziierten Genen ausgewählt ist, wobei das Verfahren einen Vorgang der Extraktion einer Nukleinsäure aus einer Probe umfasst, die eine Magenschleimhaut-Spülflüssigkeit enthält, welche durch Spülen der Magenschleimhaut erhalten wurde, die durch die Verabreichung eines Mittels zur Entfernung des Magenschleims und anschließendes Spülen und Entfernen des Magenschleims freigelegt wurde.

2. Verfahren nach Anspruch 1, des Weiteren umfassend einen Vorgang der Einstellung des pH-Wertes der Probe auf zwischen 3 und 11.

3. Verfahren zur Evaluierung der therapeutischen Wirksamkeit eines Arzneimittels und/oder eines Behandlungsverfahrens, umfassend einen Vorgang des Nachweises eines krankheitsbezogenen Markers unter Anwendung des Verfahrens nach Anspruch 1 oder 2.

4. Verwendung einer probe, die eine Magenschleimhaut-Spülflüssigkeit enthält, in dem Verfahren nach einem der Ansprüche 1 bis 3, wobei die Magenschleimhaut-Spülflüssigkeit erhalten wird durch Spülen der Magenschleimhaut, welche durch die Verabreichung eines Mittels zur Entfernung des Magenschleims und anschließendes Spülen und Entfernen des Magenschleims freigelegt wurde.

5. Verwendung nach Anspruch 4, bei welcher die Probe 5 µg oder mehr einer Nukleinsäure enthält.

6. Verwendung nach Anspruch 4 oder 5, bei welcher die Probe des Weiteren einen Chelatbildner und/oder ein Mittel zur pH-Wert-Einstellung enthält.

7. Verwendung nach einem der Ansprüche 4 bis 6, bei welcher der pH-Wert der Probe 3 bis 11 beträgt.

8. Verwendung eines Vorbehandlungsmittels, das ein Mittel zur Magenschleimentfernung und ein Mittel zur pH-Wert-Einstellung, mit welchem der pH-Wert einer Probe auf 3 bis 11 eingestellt werden kann, enthält, in dem Verfahren nach einem der Ansprüche 1 bis 3.

9. Verwendung eines Probensammelbehälters, der einen Chelatbildner und/oder ein Mittel zur pH-Wert-Einstellung enthält und der mit einer Saugleitung eines Endoskopiegerätes verbunden werden kann, in dem Verfahren nach einem der Ansprüche 1 bis 3.

## Revendications

1. Procédé de détection d'un marqueur associé à une maladie sélectionné dans le groupe constitué par un gène associé au cancer, *H. pylori* et les gènes qui lui sont associés, le VEB et les gènes qui lui sont associés, et le CMV et les gènes qui lui sont associés ; ledit procédé comprenant un processus consistant à extraire un acide nucléique d'un échantillon contenant un fluide de lavage de muqueuse gastrique obtenu en lavant la muqueuse gastrique exposée en raison de l'administration d'un agent d'élimination du mucus gastrique et en lavant et en éliminant ensuite le mucus gastrique.

2. Procédé selon la revendication 1, comprenant en outre un processus d'ajustement du pH de l'échantillon pour qu'il se situe entre 3 et 11.

3. Procédé d'évaluation de l'efficacité thérapeutique d'un médicament et/ou d'un procédé de traitement, comprenant un processus de détection d'un marqueur associé à une maladie à l'aide du procédé selon la revendication 1 ou 2.

4. Utilisation d'un échantillon contenant un fluide de lavage de muqueuse gastrique dans le procédé selon l'une quelconque des revendications 1 à 3, dans laquelle le fluide de lavage de muqueuse gastrique est obtenu en lavant la muqueuse gastrique exposée en raison de l'administration d'un agent d'élimination du mucus gastrique et en lavant et en éliminant ensuite le mucus gastrique.

5. Utilisation selon la revendication 4, dans laquelle l'échantillon contient 5 µg ou plus d'un acide nucléique.

6. Utilisation selon la revendication 4 ou 5, dans laquelle l'échantillon contient en outre un chélateur et/ou un ajusteur de pH.

7. Utilisation selon l'une quelconque des revendications 4 à 6, dans laquelle le pH de l'échantillon va de 3 à 11.

8. Utilisation d'un agent de prétraitement contenant un agent d'élimination du mucus gastrique, et un ajusteur de pH qui peut ajuster le pH d'un échantillon pour qu'il se situe entre 3 et 11, dans le procédé selon l'une quelconque des revendications 1 à 3.

9. Utilisation d'un récipient de collecte d'échantillons contenant un chélateur et/ou un ajusteur de pH et pouvant être relié à un tuyau d'aspiration d'un appareil endoscopique, dans le procédé selon l'une quelconque des revendications 1 à 3.
